# EUROPÄISCHE PATENTSCHRIFT

(11) **EP 3 287 156 B1**
(45) Veröffentlichungstag und Bekanntmachung des Hinweises auf die Patenterteilung: **10.04.2024**
(21) Anmeldenummer: 17186368.1
(22) Anmeldetag: 16.08.2017
(51) Int. Cl.: A61M 1/34

(54) **SYSTEM ZUR POST-DIALYTISCHEN BESTIMMUNG DES TROCKENGEWICHTS**
SYSTEM FOR POST-DIALYTIC DETERMINATION OF DRY WEIGHT
SYSTÈME DE DÉTERMINATION POST-DIALYTIQUE DU POIDS SÈC

(30) Priorität: 22.08.2016 DE 102016115496
(43) Veröffentlichungstag der Anmeldung: 28.02.2018
(73) Patentinhaber: B. Braun Avitum AG, 34212 Melsungen (DE)
(72) Erfinder: ATALLAH, Richard, 34125 Kassel (DE); BAUER, Florian, 51789 Lindlar (DE)
(74) Vertreter: Winter, Brandl - Partnerschaft mbB

(56) Entgegenhaltungen:
- DE-A1-102013 108 543
- US-A1- 2014 249 384
- US-B2- 8 496 807
- F Lopot ET AL: "CONTINUOUS BLOOD VOLUME MONITORING AND "DRY WEIGHT" ASSESSMENT", Journal of Renal Care, 1. April 2007 (2007-04-01), Seiten 52-58, XP055428403, Oxford, UK DOI: 10.1111/j.1755-6686.2007.tb00040.x Gefunden im Internet: URL:http://eolit-p.internal.epo.org/api/or ders/186562/pdf
- HECTOR J. RODRIGUEZ ET AL: "Assessment of dry weight by monitoring changes in blood volume during hemodialysis using Crit-Line", KIDNEY INTERNATIONAL, Bd. 68, Nr. 2, 1. August 2005 (2005-08-01) , Seiten 854-861, XP055428266, LONDON, GB ISSN: 0085-2538, DOI: 10.1111/j.1523-1755.2005.00467.x

## Beschreibung

Die vorliegende Erfindung betrifft ein nicht beanspruchtes Verfahren und ein System (bzw. eine Vorrichtung) für ein Messsystem zur Bestimmung des Trockengewichts eines Patienten während einer Dialysetherapie und nach Beendigung einer Ultrafiltration in einer Dialyseroutine. Im Besonderen befasst sich die Erfindung mit der Evaluierung des erreichten Trockengewichts bei einer Dialysetherapie.

### Hintergrund der Erfindung

Ziel einer Dialysetherapie ist es, neben der Entgiftung des Blutes, überschüssiges Wasser, welches sich aufgrund eines der Dialyse zugrundeliegenden Nierenversagens im Körper ansammelt, aus diesem zu entfernen. Dies geschieht durch eine sogenannte Ultrafiltration, bei der Flüssigkeit über einen Dialysator aus dem Blut entfernt wird. Während der sogenannten intradialytischen Zeit lagert sich Wasser zu 64 % im intrazellulären Raum, zu 28 % im Interstitium und nur zu 8% im Blut ab.

Wird während der Ultrafiltration Plasma aus dem Blut entfernt, so strömt Wasser durch Osmose aus dem intrazellulären Raum in den intravasalen Raum. Dieser Vorgang wird als "Refilling" bezeichnet. Ziel ist es, unter anderem, das überschüssige Wasser aus dem Körper eines Patienten zu entfernen, um den Patienten wieder auf sein Ausgangsgewicht ohne Überwässerung und ohne Komplikationen zu bringen. Dieses Ausgangsgewicht wird auch Trockengewicht genannt. Eine fehlerhafte Bestimmung des Trockengewichts kann zu starken Komplikationen führen. So führt bspw. ein zu niedrig bestimmtes Trockengewicht zu hohem Wasserentzug und damit zu einer Unterwässerung (Hypovolämie), was mit Hypotonie verbunden ist, wohingegen ein zu hoch bestimmtes Trockengewicht zu einer stetigen Überwässerung (Hypervolämie) führt. Sowohl Überwässerung als auch Unterwässerung des Patienten sind mit einer hohen Mortalitätsrate verbunden.

Die Trockengewichtbestimmung in der Dialyseroutine beruht herkömmlicher Weise auf Erfahrungen und Routinen des klinischen Personals, wobei z.B. die Blutdruck- und Blutvolumenverläufe der Therapien evaluiert und dementsprechend ein adäquates Trockengewicht bestimmt wird.

### Stand der Technik

Gemäß dem Stand der Technik werden zur Bestimmung bzw. Beurteilung des Trockengewichts die Ansätze der Bioimpedanz-Messung, der Blutvolumen-Überwachung, der Vena-Cava-Durchmesser-Messung und der biochemischen Marker unterschieden, die im Folgenden kurz erläutert werden.

Bei der Bioimpedanz Messung werden zwei Elektroden an den Patienten angeschlossen und zwischen ihnen ein schwacher Strom (1 mA) mit verschiedenen Frequenzen (1 kHz - 1MHz) übertragen. Aufgrund der Beschaffenheit der Zellmembran und der Flüssigkeit im Gewebe, welche nur von höheren Frequenzen durchdrungen wird, lässt sich auf Grundlage der gemessenen Impedanz die Flüssigkeit im intra- und extrazellulären Raum sehr genau bestimmen. Das Verfahren bietet Momentaufnahmen über den Wasserhaushalt der verschiedenen Reservoirs prä-, intra- und postdialytisch. Anhand dieser Werte lassen sich dann Flüssigkeitsübergänge und das postdialytische Endstadium bestimmen, welches Aufschluss über eine mögliche Über- bzw. Unterwässerung liefert.

Bei der Vena-Cava-Durchmesser-Messung (Vena Cava Diameter Measurement) wird mittels Ultraschall der Durchmesser der unteren Hohlvene bestimmt. Aufgrund der Wasseransammlung in der inter-dialytischen Zeit, sind die Venen weiter geweitet als bei normal hydrierten Personen. Der festgestellte Durchmesser der Vene wird dann mit gewissen Referenzwerten verglichen. So indiziert z.B. ein Durchmesser von 11mm/m² eine Überwässerung, wohingegen ein Durchmesser von 8 mm/m² eine Unterwässerung anzeigt.

Beim Blutvolumen-Monitoring wird der Hämatokrit-Gehalt (Hct-Gehalt, entspricht dem Anteil der Erythrozyten im Blut) des Blutes bestimmt, über den auf den Wasseranteil im Blut zurückgerechnet werden kann. So weist ein hoher Hct-Gehalt/Wert bezogen auf ein gewisses Volumen auf einen niedrigen Flüssigkeitsstand hin. Im Gegensatz hierzu indiziert ein geringer Hct-Wert eine Überwässerung. Das Blutvolumen wird während einer Therapie mit einem Hct-Sensor kontinuierlich ausgelesen und es kann anhand der so entstehenden Kurve eine Aussage über den Wasserhaushalt des Patienten getroffen werden. So hängt der Verlauf der Blutvolumen-Kurve weitestgehend von dem Verhältnis der Ultrafiltrations-Rate zu dem körpereigenen Refilling ab. Ist ein hohes Refilling und dementsprechend eine flache Blutvolumen-Kurve vorhanden, kann auf eine eventuelle Überwässerung geschlossen werden. Diese Daten werden nun von Ärzten und Personal genutzt, um anhand von Erfahrungs- und Routinewerten eine Einschätzung über das Erreichen des Trockengewichts zu treffen.

Beim Verfahren unter Verwendung von biochemischen Markern kann bspw. ein Marker für das Atrial Natriuretic Peptide (ANP) eigesetzt werden. Abhängig von den Änderungen des transmuralen Drucks (Differenz zwischen Innen- und Außendruck bei z.B. Blutgefäßen) wird ANP im Zellgewebe gebildet und ausgeschüttet. Das ANP spielt eine entscheidende Rolle für die Wasser-Salz-Homöostase und hat eine sehr geringe Dialyse Clearance (die renale Clearance gibt die Eliminierung (das "Clearing") eines Stoffes aus dem Blutplasma an). Aus diesem Grund, bietet dieser Marker eine gute Möglichkeit zur Bestimmung des körpereigenen Flüssigkeitshaushalts vor und nach der Dialyse. Es wurde festgestellt, dass die ANP-Konzentration sich während einer Dialysetherapie deutlich verändert und gute Korrelationen zum Blutdruck zeigt.

Die vorgenannten Ansätze haben sich jedoch in der Dialyseroutine nicht oder nur teilweise durchgesetzt, da sie nur Anhaltspunkte für eine Einschätzung des Trockengewichts liefern. Das Trockengewicht wird somit weiterhin meist nur über die Erfahrungswerte des Dialysepersonals bestimmt.

Ferner sind die vorgenannten Ansätze mit den folgenden Nachteilen verbunden:
Zur Messung der Bioimpedanz werden spezielle Elektroden und ein Bioimpedanz-Gerät benötigt, was mit zusätzlichen Kosten verbunden ist. Weiterhin führen das Befestigen der Elektroden und das Auswerten der Ergebnisse während bzw. nach der Therapie zu einem Mehr-Aufwand für die Belegschaft des Dialysezentrums. Zudem wird keine exakte Aussage über die genaue Menge an Über- bzw. Unterwässerung getroffen, sondern nur über den Wasseraustausch zwischen den Reservoirs und deren Füllstand. Demnach ist somit ein Arzt notwendig, um die Ergebnisse zu interpretieren, auch wenn sogenannte Shift-Vektoren bei der Auswertung unterstützen können.

Bei der Vena-Cava-Durchmesser-Methode wird der Venendurchmesser nur zu bestimmten Zeitpunkten (z.B. vor, während oder nach der Therapie) bestimmt. Einflussfaktoren, wie Vasokonstriktion oder andere Erkrankungen, die Einfluss auf den Durchmesser der Vene haben, werden nicht berücksichtigt. Ferner können Änderungen im Venendurchmesser zwischen verschiedenen Patienten starke Schwankungen haben. Dieses Verfahren benötigt weiterhin ein zusätzliches Ultraschallgerät und einen interpretierenden Arzt, was zu einem erhöhten Einsatz und Aufwand von Personal führt.

Beim Blutvolumen-Monitoring handelt es sich um kein direktes Verfahren zur Bestimmung des Trockengewichts. Dieses Verfahren liefert dem Arzt nur einen Anhaltspunkt, welchen dieser für seine persönliche Einschätzung heranzieht. Das System liefert zwar präzise Werte über den Wasseranteil, der sich aktuell und bezogen auf den Beginn der Therapie im Blut befindet, doch wird weder eine Aussage über mögliche Über- oder Unterwässerungen noch über die Wassermenge in den anderen Reservoirs getroffen. Der behandelnde Arzt muss die Blutvolumen-Werte selbst evaluieren und beruhend auf seiner Erfahrung entscheiden, ob das Trockengewicht bei weiteren Therapien erhalten bleibt oder verändert werden soll. Es handelt sich also um eine Art Trial-and-Error-Verfahren, bei dem der Arzt sich das Verhalten des Blutvolumens ansieht und daraufhin Entscheidungen bezüglich der Ultrafiltrations-Menge trifft. Aus diesem Grund liefert dieses Verfahren keine genauen Aussagen über ein zu niedrig oder zu hoch gewähltes Trockengewicht.

Schließlich ermöglicht das Verfahren unter Verwendung der biochemischen Marker die Erfassung einer Überwässerung nach der Therapie. Eine Unterwässerung kann mit diesem Verfahren aber nicht festgestellt werden. Ferner ist das Ergebnis der Überwässerung von der Herzfunktion des Patienten abhängig, da diese den Füllstand und damit auch den transmuralen Druck maßgeblich beeinflusst. Trotz guter Korrelation von Marker-Konzentration zu Blutdruck sind die Ergebnisse dieses Verfahrens gemessen an den aktuellen Standards unsicher. Ein weiterer Nachteil ist, dass diese Methode für eine Massenanwendung sehr umständlich und zeitaufwändig ist, da Laboranalysen und zusätzliche Gerätschaften sowie Personal zur Bedienung solcher zusätzlichen Gerätschaften benötigt werden. US 2014/249384 A1 beschreibt Verfahren, Vorrichtungen, Computerprogramme und Computerprogrammprodukte zum Schätzen eines Trockengewichts eines Dialysepatienten durch Bestimmen eines ersten Flüssigkeitsstatus des Patienten zwischen Behandlungssitzungen in einer ersten Stufe, Bestimmen eines zweiten Flüssigkeitsstatus des Patienten während Behandlungssitzungen in einer zweiten Stufe und Schätzen des Trockengewichts auf der Grundlage des zweiten Flüssigkeitsstatus.

US 8,496,807 B2 beschreibt eine Hämodialysevorrichtung und ein Hämodialyseverfahren, das die Hämodialyse und Ultrafiltration durch extrakorporal zirkulierendes Blut eines Patienten durchführen kann. DE 10 2013 108543 A1 beschreibt Vorrichtungen und Verfahren zur Vorhersage intradialytsicher Parameter wie z.B. eines Blutdrucks, wobei mindestens ein Lernalgorithmus und/oder ein neuronales Netz vorgesehen ist.

### Kurzbeschreibung der Erfindung

Der vorliegenden Erfindung liegt u.a. die Aufgabe zugrunde, ein System/eine Vorrichtung zur Bestimmung eines Trockengewichts eines Patienten während einer Dialysetherapie und nach Beendigung einer Ultrafiltration bereitzustellen, mittels denen eine selbsttätige und automatisierte Bewertung/Beurteilung des Trockengewichts und eine einfache Implementierung in vorhandene Systeme möglich sind.

Diese Aufgabe wird gelöst durch ein System nach Patentanspruch 1.

Dementsprechend wird erfindungsgemäß die Änderungsrate oder Steigung des sogenannten Blutvolumen-Rebounds nach Erreichen eines eingestellten/vorbestimmten Ultrafiltrations-Volumens festgestellt und mithilfe der Lerneinrichtung (z.B. neuronales Netz) wird geprüft, ob ein für einen Patienten vordefiniertes Trockengewicht erreicht wurde und, falls nicht, inwiefern das Ultrafiltrations-Volumen angepasst werden kann. Es folgt also eine selbsttätige Bewertung des Trockengewichts, im Gegensatz zu den herkömmlichen Ansätzen für mathematische oder messtechnische Verfahren, welche immer noch vom behandelnden Personal eingeschätzt und interpretiert werden müssen. Das vorgeschlagene System/Vorrichtung bzw. Verfahren zur Bestimmung des Trockengewichts lässt sich somit auf einfache Weise in schon vorhandene Systeme implementieren (sofern das herkömmliche System über ein Blutvolumen-Monitoring verfügt) und trainiert sich selbst voll automatisiert durch die Lerneinrichtung bzw. den Lernschritt.

Weiterhin lässt sich das System/die Vorrichtung bzw. das nicht beanspruchte Verfahren an individuelle Patienten anpassen und liefert am Ende jeder Therapie/Dialyseroutine eine explizite Aussage über das Erreichen des Trockengewichts sowie eventuelle Anpassungen des Ultrafiltrations-Volumens.

Somit kann also in vorteilhafter Weise eine genaue Bestimmung des Trockengewichts über den Blutvolumen-Rebound mittels einer Lerneinrichtung (z.B. neuronales Netz) erfolgen, wobei eine einfache Implementierung in vorhandene Systeme möglich ist. Die integrierte Lerneinrichtung bzw. der integrierte Lernschritt ermöglichen ein selbstständiges Lernen und eine selbstständige Optimierung der Trockengewichtbestimmung. Sie kann vollständig automatisiert werden und es sind keine zusätzlichen Messgeräte erforderlich, was somit zu keinem Sonderaufwand für Patient und Personal führt.

Im Ergebnis wird ein schnelles und direktes Verfahren bereitgestellt, das individuell auf Patienten abgestimmt werden kann und zu weniger Komplikationen durch eine genaue Bestimmung des Trockengewichts führt. Vorteilhaft ist dabei auch, dass das Verhältnis zwischen Blutvolumen und Rebound zu Hydrationszustand auch bei Änderungen im Trockengewicht unbeeinflusst bleibt.

Spezifische vorteilhafte Ausführungsformen der vorliegenden Erfindung sind in den Unteransprüchen angegeben.

### Figurenbeschreibung

Im Folgenden werden bevorzugte Ausführungsbeispiele der vorliegenden Erfindung unter Bezugnahme auf die beiliegenden Zeichnungsfiguren näher beschrieben. Es zeigen:
Fig. 1 ein Flussdiagramm eines Verfahrens zur Bestimmung des Trockengewichts gemäß einem ersten Ausführungsbeispiel;
Fig. 2 eine schematische Architektur eines neuronalen Netzwerks zur Verwendung in der vorliegenden Erfindung;
Fig. 3 eine Tabelle mit beispielhaften Ergebnissen verschiedener Durchläufe eines neuronalen Netzwerks; und
Fig. 4 eine weitere Tabelle mit beispielhaften Ergebnissen verschiedener Durchläufe verschiedener neuronaler Netzwerke mit unterschiedlichen Eingangsebenen und versteckten Ebenen.

Im Folgenden werden bevorzugte Ausführungsbeispiele der vorliegenden Erfindung am Beispiel eines lernfähigen Messsystems zur Bestimmung/Beurteilung des Trockengewichts eines Patienten beschrieben. Die im Folgenden erwähnten Verfahren fallen als solche nicht unter den beanspruchten Gegenstand und dienen lediglich einem besseren Verständnis.

Über die während und nach der Dialysetherapie aufgenommenen Blutvolumen-Werte soll dabei eine möglichst genaue Aussage über das Trockengewicht getroffen werden können. Hierbei wird der Blutvolumen-Rebound, also die Steigung des Blutvolumens nach Beendigung der Dialysetherapie, herangezogen, um eine Aussage über das Erreichen des Trockengewichts und den Grad einer eventuellen Über- bzw. Unterwässerung zu treffen. Allgemein gilt, je höher die Steigung des Blutvolumens nach der Dialysetherapie, desto größer der Grad der Überwässerung des Patienten und desto größer das noch tolerierbare Ultrafiltrations-Volumen/ die noch tolerierbare Ultrafiltrations-Menge.

Anhand einer geeigneten Verarbeitung der Blutvolumen-Werte kann dann die Steigung des Blutvolumens nach dem Beenden einer Therapiezeit (Dialysetherapie und vorbestimmte Zeit) automatisch evaluiert und eine genaue Aussage über das Erreichen des Trockengewichts getroffen werden. Hierfür werden die Blutvolumen-Werte nach Erreichen des angegebenen/vorbestimmten Ultrafiltrations-Volumens für einen gewissen Zeitraum/ vorbestimmte Zeit aufgezeichnet und mit Hilfe einer bspw. auf einem Algorithmus basierenden Lerneinrichtung (z.B. ein neuronales Netz) evaluiert. Die Lerneinrichtung berechnet/ermittelt anschließend das noch fehlende oder das überflüssige Ultrafiltrations-Volumen, sodass der Arzt seine nächsten Therapien dementsprechend anpassen kann.

Gemäß den im Folgenden beschriebenen Ausführungsbeispielen wird ein neuronales Netz als Beispiel der Lerneinrichtung bereitgestellt, welches die Steigung des Rebounds evaluiert, um das benötigte Ultrafiltrations-Volumen zum Erreichen des Trockengewichts zu bestimmen. Das neuronale Netz kann hierbei bspw. mithilfe von Daten einer (vorab durchgeführten) klinischen Studie oder anderer vorbestimmter Trainingsdaten trainiert werden. Das Training bildet somit eine Voraussetzung für die Definition eines etablierten neuronalen Netzes zur Bestimmung oder Beurteilung des Trockengewichts. Für diese Daten wird bspw. bei stabilen Dialysepatienten mit bekanntem Trockengewicht zu einem bestimmten Zeitpunkt vor Ende der Therapiezeit bei bekanntem verbleibendem Ultrafiltrations-Volumen die Ultrafiltration gestoppt und der Blutvolumen-Rebound (z.B. mit Hilfe eines in die Dialysemaschine integrierten oder externen Hämatokrit-, Toxin-, Ultraschall- oder Stoffkonzentrationssensor) für 15min aufgezeichnet. Die so erhaltenen Datenpaare werden dem Netzwerk zum Training übergeben. Das neuronale Netz lernt durch das Training, welcher Blutvolumen-Rebound welcher Volumenmenge entspricht. Im Anschluss an die Studie wird das trainierte neuronale Netzwerk in der Dialysemaschine implementiert.

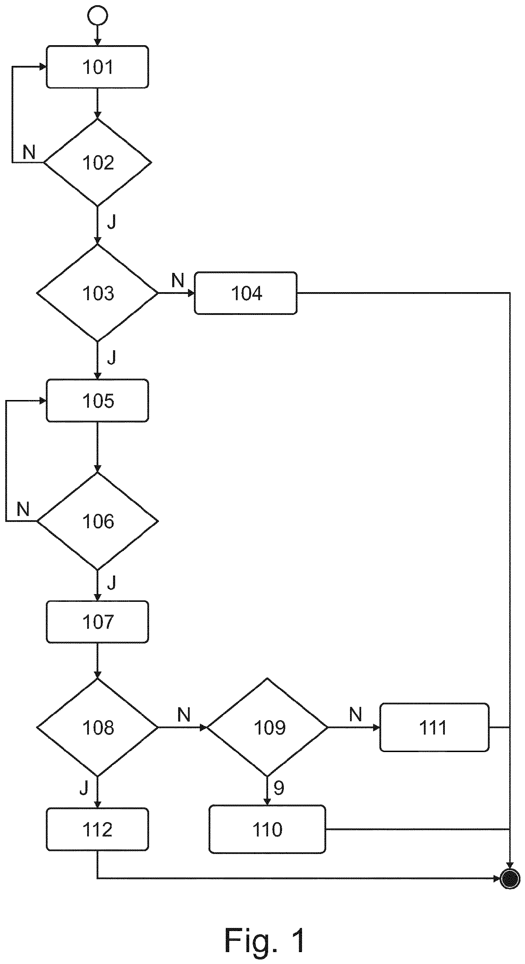

Fig. 1 zeigt ein Flussdiagramm eines (Steuerungs-)Verfahrens zur Bestimmung des Trockengewichts gemäß einem ersten Ausführungsbeispiel. Das Verfahren kann bspw. als ein Computerprogramm implementiert sein, dessen Codemittel (Instruktionen etc.) bei dessen Ausführung auf einem Computersystem die nachfolgenden Schritte generieren.

Im Schritt 101 wird eine Dialysetherapie mit Blutvolumen-Überwachung durchgeführt. Die Therapieparameter (z.B. Therapiezeit) werden so angepasst, dass das benötigte Ultrafiltrations-Volumen eine vorbestimmte Zeitdauer (z.B. 15min) vor dem eigentlichen Ende der Therapiezeit erreicht wird. Im Schritt 102 wird geprüft, ob die Therapiezeit abzüglich der vorbestimmten Zeitdauer bereits erreicht ist oder nicht. Ist die Therapiezeit Minus der vorbestimmten Zeitdauer erreicht, so schreitet der Ablauf entlang des Ja-Zweiges (J) zum Schritt 103. Falls nicht, kehrt der Ablauf entlang des Nein-Zweiges (N) zurück zum Schritt 101.

Im Schritt 103 wird dann geprüft, ob das gewünschte Ultrafiltrations-Volumen erreicht ist oder nicht. Ist das Ultrafiltrations-Volumen nicht erreicht, so schreitet der Ablauf entlang des Nein-Zweiges (N) zum Schritt 104 und die Dialysetherapie wird bis zum Erreichen des Ultrafiltrations-Volumens fortgesetzt oder beendet und das überschüssige Volumen wird bei der nächsten Therapie entzogen.

Wird dagegen im Schritt 103 festgestellt, dass das Ultrafiltrations-Volumen erreicht wurde, so schreitet der Ablauf entlang des Ja-Zweiges (J) zum Schritt 105 und die Dialyseroutine wird ohne Ultrafiltration bis zum Ende der Therapiezeit durchgeführt. Während der Restzeit der vorbestimmten Zeitdauer (z.B. 15min) wird die Blutvolumen-Überwachung fortgeführt.

Im darauffolgenden Schritt 106 wird dann geprüft, ob die Therapiezeit erreicht worden ist. Falls nicht, kehrt der Ablauf entlang des Nein-Zweiges (N) zum Schritt 105 zurück. Andernfalls schreitet der Ablauf entlang des Ja-Zweiges (J) zum Schritt 107, wo die Blutvolumenwerte, genauer gesagt der Blutvolumen-Rebound, für die Evaluierung des Trockengewichts gespeichert und vom neuronalen Netz ausgewertet werden. Nach dieser Auswertung prüft das neuronale Netz im darauffolgenden Schritt 108, ob das Trockengewicht erreicht wurde. Falls im Schritt 108 basierend auf den antrainierten Erfahrungswerten des neuronalen Netzes festgestellt wird, dass das Trockengewicht noch nicht erreicht wurde, so schreitet der Ablauf entlang des Nein-Zweiges (N) zum Schritt 109, wo geprüft wird, ob eine Über- oder Unterhydratisierung des Patienten vorliegt. Weiterhin ermittelt das neuronale Netz eine genaue Information über die Volumenmenge des Hydratationszustands. Falls im Schritt 109 eine Überhydratisierung berechnet wird, so schreitet der Ablauf entlang des Ja-Zweiges (J) zum Schritt 110, wo der Patient automatisch weiter ultrafiltriert oder ein Arzt (Systembenutzer) über die Überhydratisierung informiert wird.

Wird dagegen im Schritt 109 eine Unterhydratisierung berechnet, so schreitet der Ablauf entlang des Nein-Zweiges (N) zum Schritt 111, wo bspw. automatisch eine Infusion einer Kochsalzlösung als Bolus veranlasst oder der Arzt (Systembenutzer) über die Unterhydratisierung informiert wird.

Falls im Schritt 108 festgestellt wird, dass das Trockengewicht erreicht wurde, schreitet der Ablauf entlang des Ja-Zweiges (J) zum Schritt 112 und das Verfahren ist beendet. Die Dialyseroutine kann also beendet werden.

Gemäß dem Ausführungsbeispiel wird ein patientenindividuelles neuronales Netz bereitgestellt, welches auf den Patienten individuell angepasst werden kann. Nach dem gleichen Trainings-Prinzip, wie oben genannt, können Rebound-Ultrafiltrations-Menge-Datenpaare für den jeweiligen Patienten ermittelt und dem neuronalen Netz zum Training übergeben werden. Dies ist aber nur möglich, wenn der Patient bereits einen stabilen Zustand erreicht hat und das Trockengewicht daher weitestgehend bekannt ist, da sonst keine Aussage über das verbleibende Ultrafiltrations-Volumen getroffen werden kann. Ist dies der Fall, kann, wie auch bei der oben beschriebenen Studie, die Ultrafiltration ab einem bestimmten Zeitpunkt gestoppt und der Blutvolumen-Rebound bis zum Ende der Therapiezeit aufgezeichnet werden. Die so erhaltenen Datenpaare des individuellen Patienten werden dem System für das Training gegeben, sodass das neuronale Netz kontinuierlich in der Lage ist, präzise Aussagen über den Flüssigkeitshaushalt des jeweiligen Patienten zu liefern.

Eine weitere Variante für das patientenindividuelle neuronale Netz ist ein kontinuierliches Training, bei dem eine Eingangsinformation vom medizinischen Personal benötigt wird. So stellt das Personal ein Ultrafiltrations-Volumen ein und wartet auf den Blutvolumen-Rebound am Ende der Therapiezeit. Das System wurde zuvor mit den Daten der klinischen Studie trainiert. Der Blutvolumen-Rebound wird mithilfe dieses neuronalen Netzes evaluiert und die Bedienperson wird aufgefordert zu beurteilen, ob die angezeigte Aussage korrekt ist. Sollte dies nicht der Fall sein, wird sie gebeten, dem System den Bewässerungszustand des Patienten sowie seine eventuellen Anpassungen an das Ultrafiltrations-Volumen mitzuteilen. Diese Daten werden wiederum gespeichert und einem weiteren neuronalen Netz, dem persönlichen neuronalen Netz, für das Training zur Verfügung gestellt. So eicht sich das persönliche neuronale Netz über die Zeit und mithilfe der Bedienperson automatisch auf den jeweiligen Patienten.

Da ferner auch bei Nutzung des Netzwerks eine Bedienperson weiterhin aufgefordert werden kann, das Ergebnis zu überprüfen, kann das Netzwerk jederzeit neu angepasst und trainiert werden.

Die oben beschriebenen Lösungen versprechen eine hohe Präzision durch die individuellen Trainingsdaten. Auch wenn sich das Gewicht ändert, bleibt das Verhältnis vom Blutvolumen-Rebound zu Hydratationszustand unbeeinflusst.

Nachfolgend wird auf die Anwendbarkeit der Ausführungsbeispiele näher eingegangen. Dafür wurde ein neuronales Netz gewählt, das Blutvolumenwerte evaluiert und daraus das Trockengewicht berechnet.

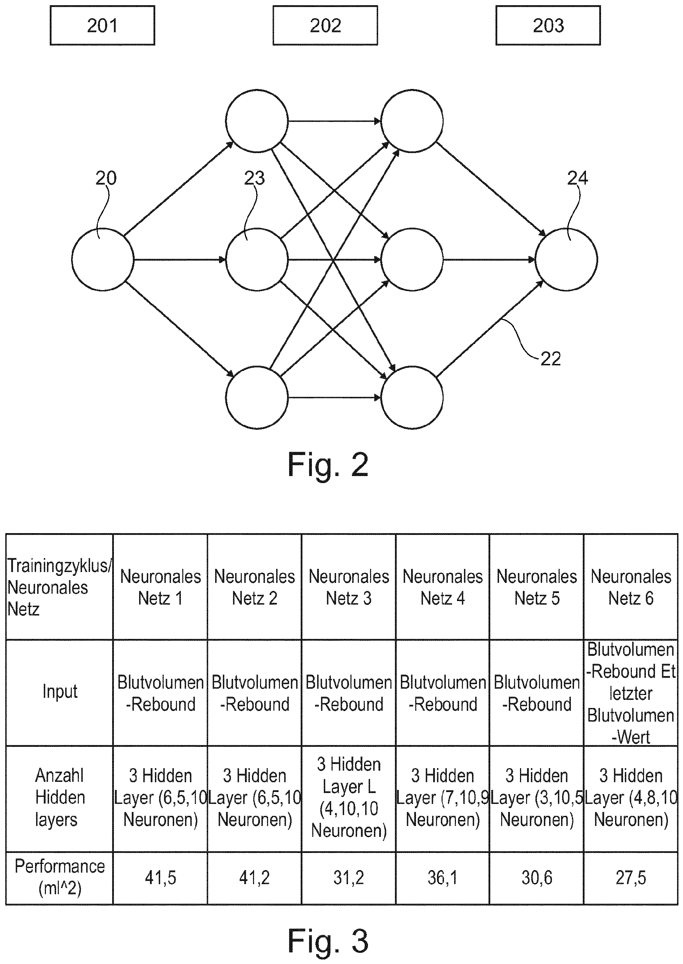

Fig. 2 zeigt eine schematische Architektur eines neuronalen Netzwerks zur Verwendung in den Ausführungsbeispielen.

Ein neuronales Netz kann als eine Softwarelösung implementiert sein, welche zur Erkennung von Mustern und Verläufen, zum Fitting von Kurven und weiteren Problemstellungen angewendet wird. Es besteht aus einer Inputschicht (Eingangsschicht) 201, welche beliebig viele Eingangsparameter 20 enthalten kann. Auf diese folgen eine oder mehrere Hiddenschichten (verdeckte Schichten) 202, welche über verschiedene Aktivierungsfunktionen (z.B. der Sigmoidfunktion) miteinander verknüpft sind. Jede Hiddenschicht 202 kann eine variable Anzahl an Neuronen 23 haben. Schließlich führt die letzte Hiddenschicht 202 auf die Output-Schicht (Ausgabeschicht) 203, welche einen oder mehrere Ausgangsparameter 24 ausgibt. Es bleibt wichtig zu erwähnen, dass je nach gewählter Anzahl von Hidden-Schichten 202 und Neuronen 23 die erwarteten Ergebnisse bzw. die Leistungsfähigkeit des Netzwerkes stark schwanken kann (die mithilfe des Mean Square Errors (MSE) zwischen berechneter und tatsächlicher Ausgabe bestimmt werden kann). Bei einer hohen Anzahl an Schichten bzw. Neuronen kann es leicht zur Überanpassung (Überfitting) kommen, wohingegen eine niedrige Anzahl nicht genug Platz zum genauen Gewichten des Outputs bietet.

Jedes neuronale Netz durchläuft zwei Schritte: Training und Validierung. Für das Training gibt es verschiedene Trainingsalgorithmen, wie z.B. das Backpropagation-Verfahren, auf welche hier allerdings nicht näher eingegangen werden soll. Beim Training werden dem Netzwerk bekannte Eingabe-Ausgabe-Paare übergeben. Das Netzwerk versucht, über die verschiedenen Aktivierungsfunktionen, mit welchen die Neuronen verknüpft sind, die erwartete Ausgabe zu erreichen. Hierbei gewichtet das neuronale Netz die verschiedenen Verbindungen 22 zwischen den Neuronen 23 der verschiedenen Schichten unterschiedlich stark, bis eine gewisse Fehlertoleranz oder eine maximale Anzahl an Trainingsdurchläufen erreicht wurde. Die Validierung erfolgt nach dem Training, indem dem Netzwerk erneut bekannte Eingabe-Ausgabe-Paare übergeben werden und es die korrekten Ausgaben mit den selbst Berechneten vergleicht.

Bei einer Ansatzvalidierung mit Hilfe von Therapiedaten wurde ein neuronales Netz mit Hilfe von 48 Therapiedaten entworfen, trainiert und validiert. Diese Therapiedaten umfassten Blutvolumen-, Ultrafiltration- sowie Blutdruck-Werte anonymer Dialysepatienten. Für das Training benötigt das neuronale Netz Blutvolumen-Rebound-Werte, die in den Therapiedaten fehlen. Aus diesem Grund, wurde der Blutvolumen-Rebound aus den einzelnen Therapiedaten wie folgt aufbereitet.

Änderungen im Blutvolumen entstehen hauptsächlich aus der Differenz von Ultrafiltration und Refilling. Mit dieser vereinfachten Betrachtung lässt sich das Refilling aus den Blutvolumen- und Ultrafiltrations-Daten der Therapien rückrechnen. Auf die Berechnung soll hier allerdings nicht weiter eingegangen werden. Das so berechnete Refilling wurde daraufhin, unter Beachtung des aus der Literatur bekannten Blutvolumen-Rebound-Verhaltens, auf den gewünschten Zeitraum nach der Therapie adaptiert (gefittet). Mit Hilfe der gefitteten Refilling-Kurven konnten dann die Blutvolumen-Rebounds berechnet werden.

Das Training erfolgte mit 30 von 48 der oben genannten Therapiedaten. Folgend darauf wurden die bestmöglichen Netzwerkparameter zur Auswertung der aufbereiteten Blutvolumen-Rebounds bestimmt. Hierfür wurden die Inputparameter verschiedenen Netzwerkkonfigurationen übergeben, trainiert und validiert. Dabei treffen jeweils drei verschiedene Eingangsparameter (Blutvolumen-Rebound, Blutvolumen-Rebound plus letzter Blutvolumen-Wert der Therapie, Blutvolumen-Werte der kompletten Therapiezeit) auf jeweils ein bis drei Hidden-Schichten mit jeweils drei bis zehn Neuronen. Jede Konfiguration wurde zehnmal trainiert. Nach Beenden des Trainingszyklus erhielt man das neuronale Netz mit der besten Performance aus bspw. 11.970 Einzeltrainings.

Für die aufbereiteten Therapiedaten wurden insgesamt sechs der oben beschriebenen Trainingszyklen durchlaufen, sodass insgesamt 71.820 Einzeltrainings stattgefunden haben.

Fig. 3 zeigt eine Tabelle mit beispielhaften Ergebnissen der besten Leistungen verschiedener Durchläufe eines neuronalen Netzwerks.

Dabei ist erkennbar, dass fünf der sechs Durchläufe den Blutvolumen-Rebound als Eingangsparameter und auf drei Hidden Layer trafen, wobei nur ein Durchlauf die beste Performance mit einem Blutvolumen-Rebound und letztem Blutvolumen-Wert als Eingangsparameter erzielte. Dieser hatte auch die beste Leistung von 27,5 ml² gezeigt. Gut zu erkennen ist allerdings, dass auch die neuronalen Netze mit einer Performance von 30,6 ml² bis 41,5 ml² sehr gute Ergebnisse lieferten. So ist die Differenz der Leistungsfähigkeit (nicht in Fig. 3 dargestellt) beim neuronalen Netz mit der schlechtesten Leistung von 41,5 ml² nur um 1 ml höher als bei dem Netzwerk mit der besten Performance von 27,5 ml². Aus diesem Grund kann nur der Blutvolumen-Rebound als Eingangsparameter in das neuronale Netz aufgenommen werden.

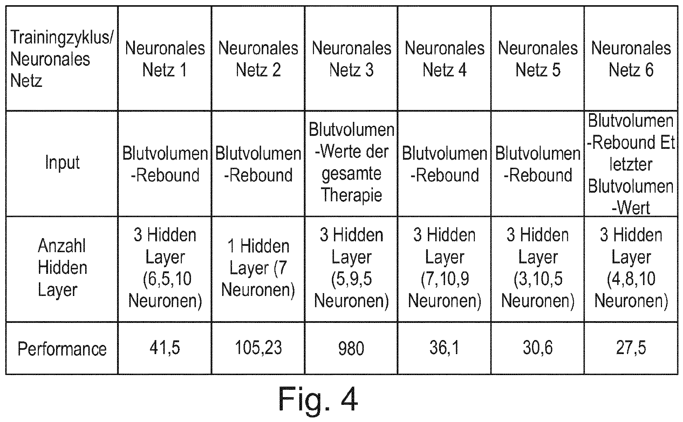

Fig. 4 zeigt eine weitere Tabelle mit beispielhaften Ergebnissen verschiedener Durchläufe verschiedener neuronaler Netzwerke mit unterschiedlichen Eingangsebenen und versteckten Ebenen.

Ein erstes neuronales Netz 1 mit Blutvolumen-Rebound als Eingangsparameter und drei Hidden Layer, ein viertes neuronales Netz 4 mit Blutvolumen-Rebound als Eingangsparameter und drei Hidden Layer, ein fünftes neuronales Netz 5 mit Blutvolumen-Rebound und letztem Blutvolumen-Wert als Eingangsparameter und drei Hidden Layer, und ein sechstes neuronales Netz 6 mit Blutvolumen-Rebound als Eingangsparameter und drei Hidden Layer liefern das neuronale Netz mit der besten Performance, wobei ein zweites neuronales Netz 2 mit Blutvolumen-Rebound als Eingangsparameter und einem Hidden Layer und ein drittes neuronales Netz 3 mit Blutvolumen-Werte der gesamten Therapiezeit als Eingangsparameter und drei Hidden Layer ein Beispiel für ein neuronales Netzes mit schlechter Leistungsfähigkeit liefern. Die Darstellung der Ergebnisse des zweiten neuronalen Netzes 2 und des dritten neuronalen Netzes 3 dienen zur Veranschaulichung, dass ein neuronales Netz mit einer gewissen Anzahl an Hidden Layer und Eingangsparametern nicht immer eine gute Leistungsfähigkeit zeigen muss.

Um die genannte Erfindung zu implementieren, sind zu Anfang Patientendaten notwendig. Diese können im Rahmen einer (vorab durchgeführten) klinischen Studie von verschiedenen Dialysezentren bezogen und aufbereitet werden. Im Rahmen dieser Studie werden stabile Patienten mit bekanntem Trockengewicht einbezogen. Bei diesen Patienten wird nun, vor Therapiezeitende, bei einer bekannten Restmenge an Ultrafiltrations-Volumen die Dialysetherapie gestoppt und der Blutvolumen-Rebound über einen bestimmten Zeitraum aufgenommen. Wenn möglich, werden auch Daten erfasst, bei denen das Ultrafiltrations-Volumen sogar definiert erhöht wird.

So können verschiedene Fälle von Über- bzw. Unterwässerung in die Trainingsdaten einfließen und das Netzwerktraining wird bessere Ergebnisse auch für Extremfälle liefern können. Die so erhaltenen Daten (Blutvolumen-Rebound und das Ultrafiltrations-Volumen) werden nun einem neuronalen Netz, dem Hauptnetzwerk, übergeben.

Diese Ergebnisse legen dann den Grundstein zur Bestimmung des Trockengewichts mit Hilfe von neuronalen Netzen.

Zusammenfassend werden ein System/eine Vorrichtung und ein nicht beanspruchtes (Steuerungs-) Verfahren zur Bestimmung des Trockengewichts eines Patienten nach einer Dialysetherapie beschrieben, wobei das Blutvolumen des Patienten überwacht und Blutvolumenwerten ausgegeben werden. Die Blutvolumenwerte werden für einen vorbestimmten Zeitraum nach Erreichen eines für den Patienten entsprechend vorbestimmten Ultrafiltrations-Volumens aufgezeichnet und ausgewertet, wobei das Trockengewicht des Patienten dann auf Grundlage der Änderungsrate des Blutvolumens während des vorbestimmten Zeitraums bestimmt wird.

## Patentansprüche

1. System zur Bestimmung eines Trockengewichts eines Patienten während einer Dialysetherapie und nach Beendigung einer Ultrafiltration, mit:
- einer Volumenmesseinrichtung, die dafür ausgestaltet/angepasst ist, das Blutvolumen des Patienten zu messen und Blutvolumenwerte auszugeben;
- einer Aufzeichnungseinrichtung, die dafür ausgestaltet/angepasst ist, die Blutvolumenwerte für einen vorbestimmten Zeitraum nach Erreichen eines für den Patienten vorbestimmten Ultrafiltrations-Volumens (102) während einer Dialysetherapie und nach Beendigung einer Ultrafiltration aufzuzeichnen; und
- einer Lerneinrichtung, die dafür ausgestaltet/angepasst ist, die aufgezeichneten Blutvolumenwerte auszuwerten und das Trockengewicht des Patienten auf Grundlage einer Änderungsrate des Blutvolumens während des vorbestimmten Zeitraums nach Erreichen eines für den Patienten vorbestimmten Ultrafiltrations-Volumens (103) während einer Dialysetherapie und nach Beendigung einer Ultrafiltration zu bestimmen (107), wobei die Lerneinrichtung ein neuronales Netz umfasst/ hat.

2. System nach Anspruch 1, wobei die Lerneinrichtung ausgestaltet/angepasst ist, eine Beendigung der Dialysetherapie einzuleiten, wenn ein für den Patienten vordefiniertes Trockengewicht erreicht wird (112).

3. System nach einem Ansprüche 1 bis 2, wobei die Lerneinrichtung ausgestaltet/angepasst ist, eine Beendigung der Dialysetherapie einzuleiten, falls das vordefinierte Trockengewicht nach Ablauf der vorbestimmten Therapiezeit nicht erreicht wird.

4. System nach einem der vorgenannten Ansprüche, wobei die Lerneinrichtung ausgestaltet/angepasst ist, eine Fortführung der Dialyseroutine ohne Ultrafiltration bis zum Ablauf einer vorbestimmten Therapiezeit einzuleiten, falls das vordefinierte Trockengewicht vor dem Ablauf der vorbestimmten Therapiezeit erreicht wurde.

5. System nach einem der vorgenannten Ansprüche, wobei die Lerneinrichtung ausgestaltet/angepasst ist, basierend auf den aufgezeichneten Blutvolumenwerten Informationen über einen Hydratationszustand des Patienten zu ermitteln.

6. System nach Anspruch 5, wobei die Lerneinrichtung ausgestaltet/angepasst ist, eine Über- oder Unterhydratisierung des Patienten zu berechnen.

7. System nach Anspruch 6, wobei die Lerneinrichtung ausgestaltet/angepasst ist, falls eine Überhydratisierung des Patienten berechnet wird, eine Fortführung der Dialysetherapie mit Ultrafiltration zu veranlassen, oder, falls eine Unterhydratisierung des Patienten berechnet wird, eine Infusion einer Kochsalzlösung als Bolus zu veranlassen.

8. System nach einem der vorgenannten Ansprüche, wobei die Lerneinrichtung ausgestaltet/angepasst ist, auf Grundlage von Datenpaaren aus Blutvolumenänderungsrate und Ultrafiltrations-Volumen anderer Patienten trainiert werden zu können.

## Claims

1. A system for determining a dry weight of a patient during a dialysis therapy and after termination of an ultrafiltration, comprising:
- a volume measuring unit configured to measure the blood volume of the patient and to output blood volume values;
- a recorder configured to record the blood volume values for a predetermined period of time after reaching an ultrafiltration volume (102) predetermined for the patient during a dialysis therapy and after termination of an ultrafiltration; and
- a learning means configured/adapted to evaluate the recorded blood volume values and to determine the dry weight of the patient based on a rate of change of the blood volume during the predetermined period of time after reaching an ultrafiltration volume (103) predetermined for the patient during a dialysis therapy and after termination of an ultrafiltration (107), the learning means comprising/including a neuronal network.

2. The system according to claim 1, wherein the learning means is configured/adapted to initiate termination of the dialysis therapy when a dry weight predefined for the patient is reached (112).

3. The system according to claim 1 or 2, wherein the learning means is configured/adapted to initiate termination of the dialysis therapy if the predefined dry weight is not reached after expiry of a predetermined therapy duration.

4. The system according to any of the preceding claims, wherein the learning means is configured/adapted to initiate continuation of the dialysis routine without ultrafiltration until expiry of a predetermined therapy duration, if the predefined dry weight has been reached before expiry of the predetermined therapy duration.

5. The system according to any of the preceding claims, wherein the learning means is configured/adapted to establish information about a hydration condition of the patient based on the recorded blood volume values.

6. The system according to claim 5, wherein the learning means is configured/adapted to calculate hyper-hydration or hypo-hydration of the patient.

7. The system according to claim 6, wherein the learning means is configured/adapted to prompt continuation of the dialysis therapy with ultrafiltration, if hyper-hydration of the patient is calculated, or to prompt infusion of a physiologic salt solution as a bolus, if hypo-hydration of the patient is calculated.

8. The system according to any of the preceding claims, wherein the learning means is configured/adapted to be trained on the basis of data pairs from the rate of change of blood volume and the ultrafiltration volume of other patients.

## Revendications

1. Système de détermination du poids sec d'un patient pendant un traitement par dialyse et à la fin d'une ultrafiltration, comprenant :
- un appareil de mesure du volume qui est conçu/adapté pour mesurer le volume sanguin du patient et fournir des valeurs de volume sanguin ;
- un appareil d'enregistrement qui est conçu/adapté pour enregistrer les valeurs de volume sanguin pendant une période prédéterminée après avoir atteint un volume d'ultrafiltration (102) prédéterminé pour le patient pendant un traitement par dialyse et à la fin d'une ultrafiltration ; et
- un appareil d'apprentissage, qui est conçu/adapté pour analyser les valeurs de volume sanguin enregistrées et pour déterminer (107) le poids sec du patient sur la base d'un taux de variation du volume sanguin pendant la période prédéterminée après avoir atteint un volume d'ultrafiltration (103) pour le patient pendant un traitement par dialyse et à la fin d'une ultrafiltration, dans lequel l'appareil d'apprentissage comprend/présente un réseau neuronal.

2. Système selon la revendication 1, dans lequel l'appareil d'apprentissage est conçu/adapté pour initier un arrêt du traitement par dialyse, lorsqu'un poids sec prédéfini pour le patient est atteint (112).

3. Système selon l'une quelconque des revendications 1 à 2, dans lequel l'appareil d'apprentissage est conçu/adapté pour initier un arrêt du traitement par dialyse, au cas où le poids sec prédéfini n'est pas atteint à la fin du temps de traitement prédéterminé.

4. Système selon l'une quelconque des revendications précédentes, dans lequel l'appareil d'apprentissage est conçu/adapté pour initier une poursuite du traitement par dialyse sans ultrafiltration jusqu'à la fin du temps de traitement prédéterminé, au cas où le poids sec prédéfini est atteint avant la fin du temps de traitement prédéterminé.

5. Système selon l'une quelconque des revendications précédentes, dans lequel l'appareil d'apprentissage est conçu/adapté pour déterminer des informations sur un état d'hydratation du patient sur la base des valeurs de volume sanguin enregistrées.

6. Système selon la revendication 5, dans lequel l'appareil d'apprentissage est conçu/adapté pour calculer une surhydratation ou une sous-hydratation du patient.

7. Système selon la revendication 6, dans lequel l'appareil d'apprentissage est conçu/adapté pour entraîner une poursuite du traitement par dialyse avec ultrafiltration, au cas où une surhydratation du patient est calculée, ou pour entraîner une perfusion de solution saline en bolus, au cas où une sous-hydratation du patient est calculée.

8. Système selon l'une quelconque des revendications précédentes, dans lequel l'appareil d'apprentissage est conçu/adapté pour pouvoir être entraîné sur la base de paires de données issues de taux de variation du volume sanguin et de volumes d'ultrafiltration d'autres patients.
